# EUROPEAN PATENT APPLICATION

(11) **EP 2 221 071 A2**
(43) Date of publication of application: **25.08.2010**
(21) Application number: 10154100.1
(22) Date of filing: 19.02.2010
(51) Int. Cl.: A61L 27/34, A61L 27/54

(54) **Medical implants having a drug delivery coating**

(30) Priority: 19.02.2009 US 389037
(71) Applicant: Biomet Manufacturing Corp., Warsaw, IN 46582 (US)
(72) Inventor: Kumar, Mukesh, Warsaw, IN 46582 (US)
(74) Representative: Gross, Felix

(57) **Abstract**

Medical implants having a drug delivery coating, comprising a diffusion matrix made of a collagen matrix, a bioactive material, and a self-arranging transport barrier layer. The bioactive material is contained in the collagen matrix layer and/or the self-arranging transport barrier layer. Methods of preparing the coated implants and methods of modulating the rate of elution of a bioactive material are also provided.

## Description

### INTRODUCTION

The present disclosure relates to medical implants having a drug delivery coating.

In various surgical procedures where a medical device is implanted into the patient, it may be advantageous to provide bioactive materials directly to the implant site. Such materials include various proteins, growth factors, drugs, nutrients, or antibiotics, as non-limiting examples. Such materials can provide a variety of benefits, including assisting in maintaining an infection free implant site, facilitating integration of the implant into the body, and preventing the need for revision surgery on the implant.

Current technologies require the use of a matrix (e.g., wax, silicone, or a film forming polymer) to adhere bioactive materials to the implant. The specific coatings are selected based on the substrate type, the bioactive materials being delivered, the type of implant and region of implantation, and ease of manufacture and storage of the coated implant, for example. Additionally, the timing of the release of the bioactive material from the coating and the quantity of the bioactive material released at a given time interval must be controlled. Current coatings and coating techniques have not sufficiently provided for such control due to limitations presented by the aforementioned selection criteria.

Moreover, current delivery technologies are also generally limited to a single platform, or they must be tailored to a specific implant type. For example, with cementless bone implants, bone will not grow into the implant where certain polymeric surfaces are employed because new bone tissue is not attracted where there is a polymer residue. The polymer coated regions in such implants do not serve as optimal binding sites for ingrowth and strong bonding of new bone.

Accordingly, there is a need for coated medical implants to effectively modulate the elution of a bioactive material. There is also a need for medical implant coatings which facilitate providing a therapy regimen and do not interfere with bone or tissue ingrowth into the medical implant. There is still further a need for simplified and uniform methods of producing coated medical implants which are applicable across a variety of platforms.

### SUMMARY

In various embodiments, the present teachings provide coated medical implants, comprising a substrate and at least two diffusion matrix layers on a surface of the substrate. Each respective diffusion matrix comprises a bioactive material, a collagen matrix layer, and a transport barrier layer adjacent to the collagen matrix layer.

In various embodiments, methods of preparing a coated medical implant are provided. A first diffusion matrix comprising a first collagen matrix, a first bioactive material, and a first transport barrier layer is applied to an implant substrate. A second diffusion matrix comprising a second collagen matrix, a second bioactive material, and a second transport barrier layer is then applied over the first diffusion matrix.

In various embodiments, the collagen matrix layer is hydrophilic or lipophilic. At least a region of the collagen matrix layer is contacted with a transport barrier material having a relative hydrophilic region and a relative lipophilic region. When the collagen matrix layer is hydrophilic, the hydrophilic region of the transport barrier material orients towards the collagen matrix layer. When the collagen matrix layer is lipophilic, the lipophilic region of the transport barrier material orients towards the collagen matrix layer.

In various embodiments, methods of modulating a rate of elution of a bioactive material from a coated medical implant to an implant site are provided. The methods comprise coating a plurality of diffusion matrix layers on the medical implant, wherein each diffusion matrix comprises a bioactive material, a collagen matrix layer, and a transport barrier layer. The medical implant is implanted into the implant site. The outermost diffusion matrix layer is contacted with a diffusion media at the implant site to release the bioactive material to the implant site at a pre-determined rate.

Further areas of applicability will become apparent from the description provided herein. It should be understood that the description and specific examples are intended for purposes of illustration only and are not intended to limit the scope of the present disclosure.

### DRAWINGS

Figure 1 depicts a stent having a coating thereon according to various embodiments;

Figure 2 depicts a cross-section of the stent of Figure 1 taken along the 2-2 line according to various embodiments;

Figure 3 depicts an acetabular cup having a coating thereon according to various embodiments;

Figures 4A-4C depict a process of coating a serrated metal surface according to various embodiments; and

Figure 5 depicts a multi-layer coating on a Copeland Shoulder according to various embodiments.

It should be noted that the figures set forth herein are intended to exemplify the general characteristics of an apparatus, materials and methods among those of this invention, for the purpose of the description of such embodiments herein. These figures may not precisely reflect the characteristics of any given embodiment, and are not necessarily intended to define or limit specific embodiments within the scope of this invention.

### DESCRIPTION

The following description of technology is merely exemplary in nature of the subject matter, manufacture and use of one or more inventions, and is not intended to limit the scope, application, or uses of any specific invention claimed in this application or in such other applications as may be filed claiming priority to this application, or patents issuing therefrom. The following definitions and non-limiting guidelines must be considered in reviewing the description of the technology set forth herein.

The headings (such as "Introduction" and "Summary") and subheadings used herein are intended only for general organization of topics within the present disclosure, and are not intended to limit the disclosure of the technology or any aspect thereof. In particular, subject matter disclosed in the "Introduction" may include novel technology and may not constitute a recitation of prior art. Subject matter disclosed in the "Summary" is not an exhaustive or complete disclosure of the entire scope of the technology or any embodiments thereof. Classification or discussion of a material within a section of this specification as having a particular utility is made for convenience, and no inference should be drawn that the material must necessarily or solely function in accordance with its classification herein when it is used in any given composition.

The description and specific examples, while indicating embodiments of the technology, are intended for purposes of illustration only and are not intended to limit the scope of the technology. Moreover, recitation of multiple embodiments having stated features is not intended to exclude other embodiments having additional features, or other embodiments incorporating different combinations of the stated features. Specific examples are provided for illustrative purposes of how to make and use the compositions and methods of this technology and, unless explicitly stated otherwise, are not intended to be a representation that given embodiments of this technology have, or have not, been made or tested.

As used herein, the words "preferred" and "preferably" refer to embodiments of the technology that afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the technology.

As referred to herein, all compositional percentages are by weight of the total composition, unless otherwise specified. As used herein, the word "include," and its variants, is intended to be non-limiting, such that recitation of items in a list is not to the exclusion of other like items that may also be useful in the materials, compositions, devices, and methods of this technology. Similarly, the terms "can" and "may" and their variants are intended to be non-limiting, such that recitation that an embodiment can or may comprise certain elements or features does not exclude other embodiments of the present technology that do not contain those elements or features.

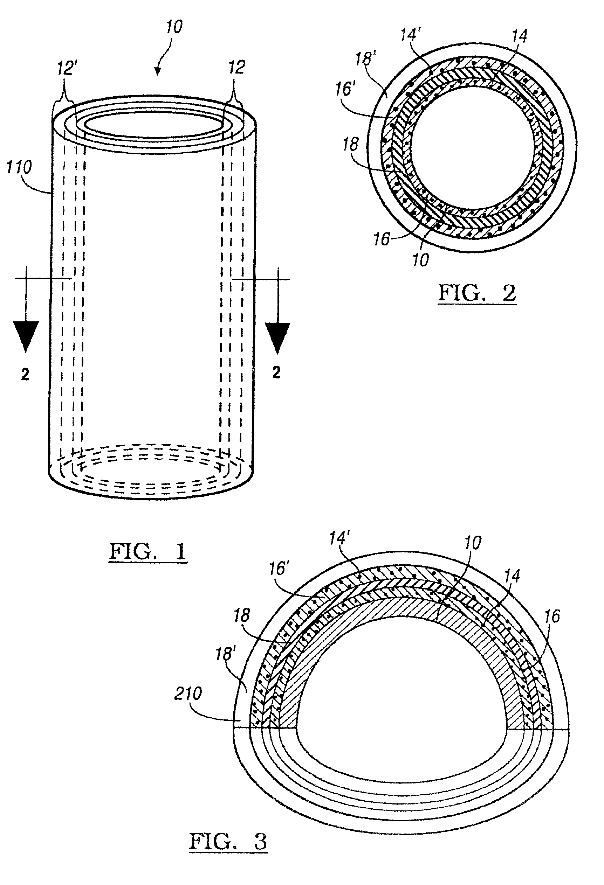

The present technology provides medical implants having at least two diffusion matrices coated thereon. For ease of discussion, Figures 1 to 5 depict various exemplary medical implant substrates 10 having a coating comprising a plurality of diffusion matrices 12, 12', 12", and/or 12"' thereon. In various embodiments, each diffusion matrix comprises a bioactive material 14, a collagen matrix layer 16, and a self-arranging transport barrier layer 18 adjacent to the collagen matrix layer 16. For clarity, the same element numbers are used for the various first and second diffusion matrices and their respective sublayers. The location and respective nature of each component is indicated using the prime notation.

It is understood that the present technology encompasses a wide variety of implants, used for a wide variety of therapeutic and cosmetic applications, in human or other animal subjects. The specific devices and materials to be used in this technology must, accordingly, be biomedically acceptable. As used herein, such a "biomedically acceptable" component is one that is suitable for use with humans and/or animals without undue adverse side effects (such as toxicity, irritation, and allergic response) commensurate with a reasonable benefit/risk ratio.

### Medical Implant Substrates

The medical implant substrate 10 can be made of any biocompatible material. Exemplary materials include stainless steel, titanium, tantalum, or another biocompatible metal, or alloys thereof; silicone, polyethylene, polypropylene, polytetrafluoroethylene, or another biocompatible polymeric material, or mixtures or copolymers thereof; polylactic acid, polyglycolic acid, or combinations thereof, or another biodegradable polymer; or mixtures or copolymers of the foregoing materials.

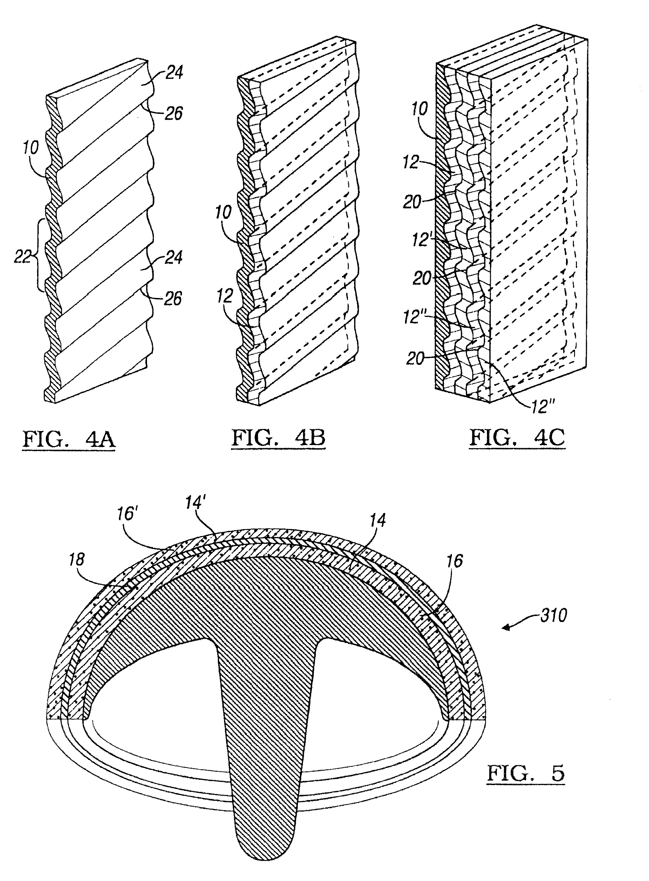

The medical implant substrate 10 can be formed as a hip, knee, elbow, shoulder, spinal, wrist, or ankle implant; a fixation plate, screw, suture anchor, and the like. Other devices can include non-orthopedic devices such as tracheotomy devices, intraurethanal and other genitourinary implants, stylets, dialators, stents, wire guides, and access ports of subcutaneously implanted vascular catheters. Although specific examples of the present disclosure relate to a stent 110 (Figure 1), acetabular cup 210 (Figure 3), and Copeland shoulder 310 (Figure 5), discussion of these medical devices are merely exemplary and not intended to limit the present teachings.

### Diffusion Matrix

At least a region of the medical implant substrate 10 is coated with at least two layers of a diffusion matrix 12 having one or more bioactive materials 14 contained therein. Generally, the first diffusion matrix 12 is coated onto the implant substrate 10 and each subsequent diffusion matrix 12 is coated over top the prior diffusion matrix 12. In the following description, only a single diffusion matrix 12 may be referred to for clarity. It is understood that the characteristics of a single diffusion matrix 12 can be employed in one or all of the other diffusion matrix layers.

In various embodiments (without limiting the function and utility of the present technology), each diffusion matrix 12 may modulate the bioactive material 14 in one or more ways. For example, the diffusion matrix 12 sublayers (collagen matrix layer 16 and self-arranging transport barrier layer 18) have regions with differing levels of resorbability or resorption to control elution of the bioactive material 14 through the various sublayers and into the adjacent environment. As used herein, the terms "resorbable" or "dissolution" and other similar terms, such as "soluble" and "degradable," and variations thereof, describe diffusion matrix sublayers that dissolve, in whole or in part, and in various embodiments lose structural integrity in a certain environment, for example, in an aqueous solution or under physiological conditions.

In various embodiments (without limiting the function or utility of the present technology), bioactive material 14 modulation is due to the polarity differences between the sublayers and polarity and water affinity differences between the aqueous solution and the sublayers which provide a rigorous and slow elution path or an easy and rapid elution path through which the bioactive material 14 can elute. The passage of the aqueous solution into the sublayers of the diffusion matrix 12 and the elution of the bioactive material 14 out of the respective sublayers of the coating is based on the relative water affinity of the sublayer and the combination of sublayers. Sublayers with a higher water affinity will provide a more rapid elution of the drug from the sublayer as compared to a sublayer with a lower water affinity. The selected arrangement of sublayers and elution of the bioactive material 14 in response to aqueous solutions can allow for sequential delivery of a regimen or therapy, as detailed later herein.

In various embodiments, the elution profile between the plurality of diffusion matrix 12 layers can overlap such that multiple bioactive materials 14 can be delivered simultaneously. In other embodiments, the elution profiles can be discrete such that each subsequent diffusion matrix 12 layer and bioactive material 14 does not elute until the prior, tissue-contacting diffusion matrix 12 layer and bioactive material elutes.

Still further, the thickness of the collagen matrix layer 16 in which the bioactive material 14 is contained also modulates the rate of bioactive material elution. A thicker collagen matrix layer 16 provides a slower elution rate and will lengthen the amount of time in which the therapy is administered. Conversely, a thinner collagen matrix layer 16 will have a relatively faster elution rate. It is understood that the thickness of the collagen matrix layers 16 can vary between the respective diffusion matrix layers 12.

The above explanations for the modulation of bioactive materials 14 are non-limiting and it is understood that combinations of other factors contribute to modulation, including selection of materials, application techniques, etc.

### Bioactive Materials

The bioactive materials 14 include any material that provides a therapeutic, nutritional, or cosmetic benefit for the human or other animal subject in which the devices of the present technology are implanted, including systemically or topically by maintaining, improving or otherwise affecting the structure or function of tissue proximate to the site at which the device is implanted. In various embodiments, such benefits include one or more of repairing of unhealthy or damaged tissue, minimizing infection at the implant site, increasing integration of healthy tissue into the medical implant, and preventing disease or defects in healthy or damaged tissue.

The bioactive material is preferably included at a safe and effective amount. A "safe and effective" amount of bioactive material is an amount that is sufficient to have the desired effect in the human or lower animal subject, without undue adverse side effects (such as toxicity, irritation, or allergic response), commensurate with a reasonable benefit/risk ratio when used in the manner of this invention. The specific safe and effective amount of the bioactive material will, obviously, vary with such factors as the particular condition being treated, the physical condition of the patient, the nature of concurrent therapy (if any), the specific bioactive material used, the specific route of administration and dosage form, the carrier employed, and the desired dosage regimen.

Bioactive materials useful in the practice of the present invention include organic molecules, proteins, peptides, peptidomimetics, nucleic acids, nucleoproteins, antisense molecules polysaccharides, glycoproteins, lipoproteins, carbohydrates and polysaccharides;, and synthetic and biologically engineered analogs thereof, living cells such as chondrocytes, bone marrow cells, viruses and virus particles, natural extracts, and combinations thereof. Specific non-limiting examples of bioactive materials include hormones, antibiotics and other antiinfective agents, hematopoietics, thrombopoietics, agents, antidementia agents, antiviral agents, antitumoral agents (chemotherapeutic agents), antipyretics, analgesics, antiinflammatory agents, antiulcer agents, antiallergic agents, antidepressants, psychotropic agents, anti-parkinsonian agents, cardiotonics, antiarrythmic agents, vasodilators, antihypertensive agents, diuretics, antichloinergics, antidiabetic agents, anticoagulants, cholesterol lowering agents, gastrointestinal agents, muscle relaxants, therapeutic agents for osteoporosis, enzymes, vaccines, immunological agents and adjuvants, cytokines, growth factors, cellular attractants and attachment agents, gene regulators, vitamins, minerals and other nutritionals, and combinations thereof.

Various embodiments of can include one or more growth factors selected from VEGF-1, a fibroblast growth factor (FGF) such as FGF-2, epidermal growth factor (EGF), an insulin-like growth factor-1 (IGF) such as IGF-1or IGF-11, a transforming growth factor (TGF) such as TGF-β, platelet-derived growth factor (PDGF), EGM, and a bone morphogenetic protein (BMP) such as BMP-2, BMP-4, BMP-6 or BMP-7.

In embodiments employing antibiotics, the antibiotics (or antimicrobial) agents are effective in preventing or inhibiting the growth of bacterial and/or fungal organisms. The term "bacterial and fungal organisms" (or bacteria or fungi) as used herein refers to all genuses and species of bacteria and fungi, including all spherical, rod-shaped, and spiral bacteria. Some examples of bacteria are *staphylococci* (i.e. *Staphylococcus epidermidis, Staphylococcus aureus*), *Enterrococcus faecalis, Pseudomonas aeruginosa, Escherichia coli,* other gram-positive bacteria and gram-negative bacilli. One example of a fungus is *Candida albicans.*

Antibiotics include the chemicals produced by one organism that are effective to inhibit the growth of another organism and include semi-synthetics, and synthetics thereof Antibiotics useful herein include macrolides and lincosamines, quinolones and fluoroquinolones, carbepenems, monobactams, aminoglycosides, glycopeptides, tetracyclines, sulfonamides, rifampins, oxazolidonones, and streptogramins, nitrofurans, derivatives thereof, and combinations thereof. Example macrolides and lincosamines include azithromycin, clarithromycin, clindamycin, dirithromycin, erythromycin, lincomycin, and troleandomycin. Example quinolones and fluoroquinolones include cinoxacin, ciprofloxacin, enoxacin, gatifloxacin, grepafloxacin, levofloxacin, lomefloxacin, moxifloxacin, nalidixic acid, norfloxacin, ofloxacin, sparfloxacin, trovafloxacin, oxolinic acid, gemifloxacin, and perfloxacin. Example Carbepenems include imipenem-cilastatin and meropenem. Example monobactams include aztreonam. Example aminoglycosides include amikacin, gentamicin, kanamycin, neomycin, netilmicin, streptomycin, tobramycin, and paromomycin. Example glycopeptides include teicoplanin and vancomycin. Example tetracyclines include demeclocycline, doxycycline, methacycline, minocycline, oxytetracycline, tetracycline, and chlotetracycline. Example Sulfonamides include mafenide, silver sulfadizine, sulfacetamide, sulfadiazine, sulfamethoxazole, sulfasalazine, sulfisoxazole, trimethoprim-sulfamethoxazole, and sulfamethizole. An example oxazolidonone is linezolid. An example streptogramin is quinupristin+dalfopristin. Other suitable antibiotics include bacitracin, chloramphenicol, colistemetate, fosfomycin, isoniazid, methenamine, metronidazol, mupirocin, nitrofurantoin, nitrofurazone, novobiocin, polymyxin B, spectinomycin, trimethoprim, colitis, cycloserine, capreomycin, ethionamide, pyrazinamide, para-aminosalicyclic acid, and erythromycin ethylsuccinate+sulfisoxazole. Still further antibiotics may also include the ample spectrum penicillins, penicillins and beta lactamase inhibitors, and cephalosporins. The antibiotics may be used alone or in combination.

In various embodiments, the diffusion matrix 12 comprises a tetracycline, a rifampin, or mixtures thereof, for example a combination of minocycline, and rifampin. Minocycline is primarily bacteriostatic and inhibits protein synthesis within a wide range of gram-positive and gram-negative organisms. Rifampin inhibits bacterial DNA-dependent RNA polymerase activity within a both gram-positive and gram-negative organisms. The combination can advantageously deter or inhibit the growth of a variety of organisms.

The amount of antibiotic in the diffusion matrix 12 is preferably an amount sufficient to provide local antimicrobial activity after elution of the antibiotic into the tissues adjacent to the implant. The "amount sufficient to provide local antimicrobial activity" refers to the sufficient amount of the antibiotic to decrease, prevent or inhibit the growth of bacterial and/or fungal organisms. The amount can vary for each antibiotic upon known factors such as pharmaceutical characteristics, the type of medical device, age, sex, health, and weight of the recipient, and the particular implant.

The bioactive material can be incorporated into either or both of the collagen layer 16 and the transport barrier layer 18. Further, different bioactive materials 14 can be incorporated into the different layers. For example, the first diffusion matrix 12 can contain a collagen matrix layer 16 with a first bioactive material 14 being protected by a first transport barrier layer 18, while the second diffusion matrix 12' can contain a collagen matrix layer 16' with a second bioactive material 14' being protected by a second transport barrier layer 18'. Further, the collagen layers 16 of the respective diffusion matrix 12 layers may contain one or more bioactive materials which are the same as, or different than, bioactive materials in other layers. Multiple bioactive materials 14 can also be incorporated into the respective diffusion matrix 12 layers.

The bioactive material 14 is incorporated into the collagen matrix layer 16 by dissolving or dispersing the bioactive material 14 in the collagen dispersion. The bioactive material and collagen dispersion can be mixed until a homogenous mixture is provided or until the dispersion has properties (viscosity, for example) to facilitate the particular application of the collagen matrix layer 16 to the medical implant substrate 10.

### Collagen Matrix Layer

The collagen matrix layer 16 is a solution or dispersion of collagen which has been applied to at least a region of the medical implant substrate 10. The collagen dispersion can be made of any collagen or collagen derivative. Collagen's basic structure consists of three polypeptide chains, each with a repeating primary amino acid sequence of -glycine-X-Y-. The collagen may be in a polymerized fibrous form that has a long three-dimensional architecture with multiple cross-links. In various embodiments, the collagen component can be fibrillar collagen, atelopeptide collagen, telopeptide collagen or tropocollagen and can be collected from a variety of mammalian or other animal sources, including human, bovine, porcine, and avian sources. Specific tissues from which collagen is derived may be mineralized or unmineralized, including from bone, tendons, skin. In some embodiments, the collagen carrier can be purified fibrillar bovine tendon Type I collagen. The collagen can be human collagen. For example, the collagen may be selected from the group comprising human Type I, II, III or IV, bovine Type I collagen, and porcine Type I collagen. Preferably, the collagen is such that there is no adverse reaction with the subject in which the collagen is used, or side reaction between the collagen in the dispersion and bioactive material or any other material of the compositions of this technology.

The collagen dispersion generally has a neutral charge. However, it may be rendered "hydrophilic" or "lipophilic" based on the presence of other materials. The bioactive materials 14, in particular, may contribute to the polarity or charge of the collagen matrix layer 16.

As is well known in the art, the terms "hydrophilic" and "hydrophobic" or "lipophilic" are relative terms. Generally, hydrophilic compounds include polar or charged moieties and have a greater solubility in aqueous solutions. Hydrophobic or lipophilic compounds include non-polar moieties and have a greater solubility in oils, for example. Still other compounds are amphiphilic. A parameter commonly used to characterize the relative hydrophilicity and lipophilicity of various compounds is the hydrophilic-lipophilic balance ("HLB" value). Generally, hydrophilic materials have an HLB value greater than about 10 while hydrophobic materials have an HLB value less than about 10.

If the HLB of a sublayer or component thereof (e.g., collagen layer 16) is lower than a reference material, then that sublayer is classified as lipophilic. If the HLB of the sublayer or component is higher than the reference material, then that layer is classified as hydrophilic. As an example of the relative assessment of hydrophilic or lipophilic classification, an exemplary first layer having an HLB value of 15 would be considered lipophilic as compared to an exemplary second layer having an HLB value of 20, although both the first layer and the second layer would be categorized as lipophilic according to the classic and non-relative HLB parameters.

### Self-Arranging Transport Barrier Layer

In various embodiments, a self-arranging transport barrier layer 18 is located next to the collagen matrix layer 16 for each respective diffusion matrix 12 layer. The self-arranging transport barrier layer 18 is made of materials that include a lipophilic region at a first end and a hydrophilic region at a second end, making the self-arranging transport barrier layer amphiphilic. The hydrophilic region and lipophilic region repel each other. The self-arranging transport barrier materials align with the adjacent layer in a pattern similar to the aggregation pattern of a micelle. In a polar or non-polar environment, the lipophilic or hydrophilic region will self-direct or be attracted to align with the polarity of the solution and maximize distance between the opposing polarity region of the self-arranging transport barrier material. For example, where the adjacent collagen matrix layer 16 is hydrophilic, the hydrophilic region of the self-arranging transport barrier layer 18 will contact the collagen matrix layer 16 and the hydrophobic tail region will remain extended out and away from the collagen matrix layer 16. The lipophilic and hydrophilic regions make the transport barrier layer 18 automatically orient or "self arrange" with respect to the collagen matrix layer 16 due to the ability to attach the lipophilic end or the hydrophilic end to the collagen matrix layer 16 based on the charge of the collagen matrix layer 16.

In various embodiments, the self-arranging transport barrier layer 18 material is a glyceryl ester. Glyceryl esters useful herein include phospholipids, derivatives thereof, salts thereof, and the like. In some embodiments, the self-arranging transport barrier layer 18 comprises lecithin. As used herein, "lecithin" includes natural, synthetic, semi-synthetic, esters and other derivatives thereof, and combinations thereof.

In addition to the self-arranging transport barrier material having a placement direction based on the adjacent layer, the transport barrier aspect of the material can expedite, hinder, or prevent the elution of a bioactive material 14 through the diffusion matrix 12. For example, when a bioactive material 14 in the collagen matrix layer 16 is completely soluble in water (or is highly hydrophilic), the highly hydrophilic bioactive material 14 may remain in the diffusion matrix 12 for an extended time because of the time required for the aqueous solution to breach the transport barrier layer 18 and pass the lipophilic region and for the bioactive material to elute back through the lipophilic region and into the surrounding environment.

### Asperities

Referring to Figures 4A through 4C, in various embodiments, asperities 20 are included between the several diffusion matrix layers. In some embodiments, these asperities 20 provide a physical barrier, such as an "air gap," between the diffusion matrix 12 layers. Such asperities may provide a discontinuous area of elution of the bioactive material 14. In some embodiments, the asperities 20 prevent the immediate elution of the bioactive material 14 by providing an additional distance through which the bioactive material 14 must elute and an additional distance until a subsequent diffusion matrix layer 12 is breached. The asperities can be a surface irregularity such as serrations, etching, grooves, channels, and the like. The asperities 20 can be of any shape including rounded, smooth, jagged, or blunt.

The metal implant substrate 10 depicted in Figures 4A through 4C shows exaggerated plasma etched serrations 22. The serrations 22 provide varying attachment regions for the plurality of diffusion matrix 12 layers to attach to the implant substrate 10. The serrations 22 have protruding regions 24 and recessed regions 26. The protruding regions 24 generally have a higher profile than the recessed regions 26 to provide texture or surface features to the metal implant substrate 10. The protruding regions 24 and the recessed regions 26 can be of any shape and are not necessarily limited to protrusions or recesses formed by plasma etching and can include the grooves, channels, ridges, etc, indicated above.

Turning to Figure 4B, a diffusion matrix 12 layer is applied over the irregular surface of the implant substrate 10. The first diffusion matrix 12 layer generally follows the contours of the surface irregularities. Figure 4C depicts a build up of a first layer 12, a second layer 12', and a third layer 12" in which the respective diffusion matrix layers have filled in the surface irregularities to provide a relatively smooth or flush diffusion matrix 12. It is understood that depending on the depth of the recessed regions 26, multiple layers of varied thicknesses could be required to provide a smooth or flush diffusion matrix 12. In still other various embodiments, it may be useful to leave a textured diffusion matrix 12.

The bioactive materials located in the recessed regions 26 nearest the serrations of the implant substrate 10 have a longer elution time than the bioactive materials located on the protruding regions 24, even within the same diffusion matrix 12 layer. This intra-layer gradient alters the elution time such that a bioactive material 14 in the protruding region 24 can elute from the diffusion matrix 12 at the same time as a bioactive material 14 located in the recessed region 26 of a second diffusion matrix 12" layer adj acent to the first diffusion matrix layer 12'.

The asperities 20 between the respective diffusion matrix 12 layers and the surface features allow for partial or limited water infiltration into the diffusion matrix 12. Additionally, the hydrophilic and lipophilic arrangement of the transport barrier layer 18 materials provide a slower or more rigorous path of elution of the bioactive material 14. The rigorous path is due to the lipophilic region or the hydrophilic region extending from a protruding region 24 of the first layer 28 and into a recessed region 26 of the adjacent second layer 30. Accordingly, the amount of time to completely breach the respective transport barrier layer is increased.

### Resorption Rates

In various embodiments, the diffusion matrices 12 of the present teachings have different resorption capabilities. For example, a combination of layers having different polarities and resorption rates can allow the bioactive material 14 to have a rapid, medium, or slow dissolution from the respective diffusion matrix 12.

As used herein, "rapid dissolution" describes coatings or subcomponents thereof that dissolve in a time period generally ranging from one minute to one week. "Medium dissolution" describes coatings or subcomponents thereof that dissolve or degrade in a time period ranging generally from one week to twelve weeks. "Slow dissolution" describes coatings or subcomponents thereof that dissolve or degrade in a time period ranging generally from twelve weeks to two years. "Stable" or "nondegradable" coatings or subcomponents thereof remain intact for longer than two years.

For example, the timing of bioactive material 14 elution from the diffusion matrix 12 can be tied to the various physiological processes and tissue remodeling at the implant site. The bioactive materials 14 can be modulated such that an antibiotic is delivered during days one through ten, various growth factors suitable for tissue remodeling (such as re-vascularization) are delivered during days 11 through 90 using two medium dissolution sublayers, and a vitamin is delivered during days 91 to 92 using a single rapid dissolution sublayer.

The addition of subsequent diffusion matrix layers, for example 12', 12", and 12'" as shown in Figure 4C, modulates the rate of elution of the totality of bioactive materials. As a non-limiting example, as shown in Figure 4C, the transport barrier 18 of the outermost diffusion matrix layer 12'" would first be breached to allow the bioactive material contained therein to elute. After elution of the bioactive material contained in the outermost diffusion matrix layer 12"', the transport barrier 18 of the subsequent diffusion matrix layer 12" would then be breached to allow the next bioactive material to elute from the system. The degradation of subsequent layers would continue in turn until the implant substrate 10 was in direct contact with the adjacent tissue.

The diffusion matrix 12 can be non-resorbable, partially resorbable, or fully resorbable. "Non-resorbable" refers to a sublayer which remains substantially intact and degrades from about 0% to about 5%. "Partially resorbable" refers to a sublayer which degrades and loses structural integrity of about 5% to about 99% of the sublayer. "Fully resorbable" refers to a sublayer which completely degrades (100%) and the sublayer is completely dissolved and absorbed by the body. In various embodiments, the diffusion matrix 12 is sufficiently resorbable to allow for bony tissue ingrowth. In some embodiments, the ingrowth is from about 100% to about 5%.

### Methods of Preparing a Medical Implant

Applying the collagen matrix layer 16 and/or the self-arranging transport barrier layer 18 to the implant substrate can be achieved using any suitable method that does not impact the effectiveness or activity of the bioactive material 14. Suitable application techniques include spraying, dipping, or spreading a solution or dispersion of the collagen matrix or the transport barrier materials, respectively, over at least a region of the substrate. The solution is maintained at a temperature sufficient to facilitate the particular application process. Suitable temperatures may be from about 10°C to about 75°C. The application of the solution should generally be an even application to facilitate adherence of the collagen matrix, transport barrier materials, and respective bioactive material 14 to the implant substrate 10 and to prevent unintentional removal thereof. The sublayers can be applied to have a substantially uniform thickness, a thickness gradient, or a variety of thicknesses spanning the surface of the substrate due to surface features on the implant or the particular application technique(s) used.

After applying each sublayer of the diffusion matrix 12, the implant 10 can be dried. Suitable drying techniques include air drying or oven drying. In embodiments where a drying oven is employed, it is desirable that the drying temperature be at a sufficiently low temperature to prevent denaturing or structural changes of the bioactive material 14. The drying may take a few seconds (from about two seconds to about 45 seconds), a few minutes (from about two minutes to about 45 minutes), or a few hours (from about one hour to about five hours). For example, in an embodiment where a dispersion of collagen and an antibiotic contains a very low concentration of the antibiotic and collagen, the drying time will generally be shorter than a dispersion having a higher concentration of the antibiotic and collagen.

When applying the self-arranging transport barrier layer 18, the hydrophilic region or lipophilic region of the transport barrier material orient towards the adjacent layer based on whether the adjacent collagen matrix layer 16 is classified as hydrophilic or lipophilic. When the collagen matrix layer 16 is classified as hydrophilic, the coating application of the self-arranging transport barrier layer 18 causes the hydrophilic region of the self-arranging transport barrier layer 18 to self-direct towards the hydrophilic collagen matrix layer 16 to protect the lipophilic ends of the self-arranging transport barrier material from being in close proximity to the hydrophilic collagen matrix layer 16. When the collagen matrix layer 16 is classified as lipophilic, the coating application of the self-arranging transport barrier layer 18 causes the lipophilic region of the self-arranging transport barrier layer 18 to self-direct towards the lipophilic collagen matrix layer 16 to protect the hydrophilic region of the self-arranging transport barrier material.

### Methods of Modulating Release of a Bioactive Material

The present technology also provides methods of administering a bioactive material to an implant site, comprising:
coating a plurality of diffusion matrix layers on a medical implant, each diffusion matrix layer comprising:
   a bioactive material;
   a collagen matrix layer; and
   a transport barrier layer,
implanting the medical implant in the implant site;
contacting the coated implant with a diffusion media at the implant site to release the at least one bioactive material to the implant site at a pre-determined rate.
In various embodiments, such methods include modulating a rate of elution of a bioactive material 14 from a coated medical implant to an implant site.

After making the necessary surgical incisions and preparing the implant area, the implant is inserted. Referring to the Copeland shoulder 310 depicted in Figure 5, contacting the Copeland shoulder 310 with the surrounding fluids in the implant site causes degradation of the sublayer and the subsequent release of the bioactive material 14 from the diffusion matrix 12 to disperse the bioactive material 14 to the surrounding tissue (localized delivery). Surrounding fluids include endogenous blood from the patient. The fluids may also be provided exogenously, such as by flushing the implant area containing the coated implant with a saline solution or sterile water. The exogenous fluid may also include previously harvested blood from the patient or any blood product, including platelet concentrate. The rapidly dissolving layers degrade first and the medium and slowly dissolving layers degrade at specific time intervals thereafter.

Referring to Figure 3, an exemplary acetabular cup 210 is coated with a diffusion matrix 12 having an antibiotic therein to reduce infection. The coated acetabular cup 210 provides localized antibiotic activity at the time of implantation, and the acetabular cup 210 implant is protected from either direct or airborne contamination of the wound. The acetabular cup 210 is also protected from an adjacent infection, such as a bacterial colonization at the wound closure. Additionally, the implant is protected from any bacteremia or bacteria in the blood which may harbor at the implant site. Providing the localized antibiotic activity reduces, inhibits, and/or prevents the growth or transmission of foreign organisms in the patient. The even coating of the layer ensures that the antibiotic activity is dispersed throughout the implant region and is not limited to a single region of the implant.

The embodiments described herein are exemplary and not intended to be limiting in describing the full scope of compositions and methods of the present technology. Equivalent changes, modifications and variations of embodiments, materials, compositions and methods can be made within the scope of the present technology, with substantially similar results.

## Claims

1. A coated medical implant, comprising:
a substrate; and
a first diffusion matrix on a surface of the substrate, the first diffusion
matrix comprising:
a first bioactive material;
a first collagen matrix layer; and
a first transport barrier layer adjacent to the collagen matrix layer;
and
a second diffusion matrix atop the first diffusion matrix, the second diffusion matrix comprising:
a second bioactive material;
a second collagen matrix layer; and
a second transport barrier layer adjacent to the collagen matrix layer.

2. A coated medical implant according to Claim 1, wherein the first bioactive material is dispersed in the first collagen matrix layer and the second bioactive material is dispersed in the second collagen matrix layer.

3. A coated medical implant according to Claim 1, wherein at least one of the first transport barrier layer or the second transport barrier layer is amphiphilic.

4. A coated implant according to Claim 1, wherein at least one of the first diffusion matrix and the second diffusion matrix is at least partially resorbable.

5. A coated implant according to Claim 1, wherein at least one of the first diffusion matrix and the second diffusion matrix is fully resorbable.

6. A coated implant according to Claim 1, wherein the first diffusion matrix has a first resorption rate and the second diffusion matrix has a second resorption rate.

7. A coated implant according to Claim 1, wherein the collagen matrix layer of the first diffusion matrix is adjacent to the implant substrate.

8. A coated medical implant, according to Claim 1, wherein the substrate is a metal substrate.

9. A coated medical implant, according to Claim 1, wherein the substrate includes a plurality of surface features, preferably wherein the substrate is plasma serrated.

10. A coated implant according to Claim 9, wherein the first diffusion matrix overlies the plurality of surface features to provide asperities between the first diffusion matrix and the second diffusion matrix.

11. A coated implant according to Claim 10, wherein the asperity has a depth equal to a thickness of at least one of the collagen matrix layer or the transport barrier layer.

12. A coated implant according to Claim 1, wherein the bioactive material is selected from the group consisting of antibiotics, drugs, growth factors, vitamins, nutrients, and combinations thereof, preferably an antibiotic.

13. A coated medical implant, according to Claim 1, wherein at least one of the collagen matrix layers contains a first bioactive material and at least one of the transport barrier layers contains a second bioactive material.

14. A coated medical implant, comprising:
a substrate;
a plurality of diffusion matrices on a surface of the substrate, each diffusion matrix comprising:
a bioactive material; and
a plurality of collagen matrix layers;
wherein each of the plurality of diffusion matrices is separated from an adjacent diffusion matrix by an asperity.

15. A coated medical implant according to Claim 14, wherein each diffusion matrix further comprises a transport barrier layer located between the collagen matrix layers.

16. A coated medical implant according to either Claim 3 or 15, wherein at least one transport barrier layer comprises a glyceryl ester, preferably lecithin.

17. A method of administering a bioactive material to an implant site, comprising:
coating a plurality of diffusion matrix layers on a medical implant, each diffusion matrix layer comprising:
a bioactive material;
a collagen matrix layer; and
a transport barrier layer,
implanting the medical implant in the implant site;
contacting the coated implant with a diffusion media at the implant site to release the at least one bioactive material to the implant site at a pre-determined rate.

18. A method according to Claim 17, wherein the plurality of diffusion matrix layers is sufficiently resorbable to allow for bony tissue ingrowth.

19. A method according to Claim 17, wherein at least one transport barrier layer comprises lecithin.

20. A method according to Claim 17, wherein the diffusion media comprises an ambient fluid at the implant site.
